# EUROPEAN PATENT APPLICATION

(11) **EP 4 154 913 A1**
(43) Date of publication of application: **29.03.2023**
(21) Application number: 21199346.4
(22) Date of filing: 28.09.2021
(51) Int. Cl.: A61K 47/64, A61P 3/10, A61P 3/00

(54) **CONJUGATES OF GLUCAGON AND AMPK ACTIVATORS**

(71) Applicant: Københavns Universitet, 1165 Copenhagen K (DK)
(72) Inventor: CLEMMENSEN, Christoffer, 1165 Copenhagen K (DK); KLEIN, Anders Bue, 1165 Copenhagen K (DK); PETERSEN, Jonas Odgaard, 1165 Copenhagen K (DK)
(74) Representative: AWA Denmark A/S

(57) **Abstract**

The present invention relates to a conjugated molecule comprising a peptide displaying at least 0.1 % activity of native glucagon at the glucagon receptor, and a AMP-activated protein kinase (AMPK) activator, the peptide being covalently bonded to the AMPK activator either directly or through a linker, the conjugated molecule for use in therapy, a pharmaceutical composition comprising the conjugated molecule, a method of reducing body weight of a mammal comprising administering the conjugated molecule to the mammal, and a non-therapeutic method of reducing body weight of a mammal comprising orally administering the conjugated molecule to the mammal.

## Description

### Technical Field

The present invention relates generally to the field of therapeutic conjugates and more specifically to conjugates having glucagon receptor activity and an AMP-activated protein kinase (AMPK) activator.

### Background Art

Metabolic disorders are any of the diseases or disorders that disrupt normal metabolism, the process of converting food to energy on a cellular level. The development of a metabolic disorder may be caused by different deficiencies such as for example genetic (inherited) deficiencies, deficiencies in certain hormones or enzymes or consumption of too much of certain foods. If left untreated, the metabolic disorders may progress into several life-threatening diseases, including but not limited to fatty liver diseases, such as Non-Alcoholic Steatohepatitis (NASH), type I or II diabetes, heart diseases, dyslipidemia and obesity.

Obesity is the most prevalent nutritional disease of humans and domestic animals such as dogs and cats in affluent societies, exceeding by far the number of nutritional deficiency diseases. As alternatives to bariatric surgery, attempts have been made to design weight-lowering drugs for the treatment of obesity. This has resulted in drugs that act by preventing the absorption of fats by acting as lipase inhibitors in the gut, or by inhibiting food intake via selective serotonin receptor 2C agonism in the hypothalamus.

Non-Alcoholic Steatohepatitis (NASH) is the second stage of non-alcoholic fatty liver diseases (NAFLD) and occurs when the build-up of fat in the liver cells is accompanied with inflammation. If left untreated, NASH progresses into fibrosis and further into cirrhosis, culminating in the need for a liver transplant. NASH is heavily influenced by lifestyle, e.g., chronic excessive calorie intake and sedentary activity, and is distinct from other fatty liver diseases caused by alcohol abuse or medication side effects. To date, no drug therapy has been FDA-approved for NASH and thus weight loss and a change in lifestyle remains the first line treatment. In response to a growing need for new treatment regimens to NASH, several NASH drugs are being developed and tested, e.g. obeticholic acid, Elafibranor and Aramchol. However, since these drugs have been associated with adverse effects, including liver toxicity and a rise in LDL cholesterol, there remains a need for drugs suitable for treatment of NASH.

Glucagon is a 29 amino acid peptide hormone which is derived from the tissue-specific posttranslational processing of the proglucagon peptide. Glucagon is predominantly secreted from the alpha cells of the pancreas and has a proven role in glucose metabolism, lipolysis, ketogenesis, energy expenditure, appetite and food intake. Glucagon offers many benefits for metabolic diseases independent from its glycemic effects, including lowering lipids in circulation and in the liver. The use of glucagon for treatment of metabolic diseases is known from the prior art. US 2020352900 A1 describes the use of hormone peptides, including glucagon, in the treatment of various disorders associated with insulin resistance and obesity. US 2006014670 A1 describes the use of glucagon and insulin to achieve therapeutically effective control of diabetes but also to prevent hypoglycemia in diabetic patients. However, the use of glucagon in treatment of metabolic diseases is halted by the effect of glucagon on increasing blood glucose levels.

AMP-activated protein kinase (AMPK) is a central regulator of energy homeostasis, which coordinates metabolic pathways and thus balances nutrient supply with energy demand. Due to the favourable physiological outcomes of AMPK activation on metabolism, AMPK has been considered to be an important therapeutic target for controlling human diseases including metabolic syndrome and cancer. In recent years, several companies have undertaken medicinal chemistry campaigns on developing AMPK activators, thus AMPK activators are known from the prior art. A list of recently identified indirect and direct AMPK activators is provided in Steinberg et al. (Nat Rev Drug Dis 2019; 18(7): 527-55141). Myers et al. (Science 2017; 357(6350): 507-511) describes an orally available AMPK activator, (3R,3aR,6R,6aR)-6-((6-([1,1'-biphenyl]-4-yl)-7-chloro-3*H*-imidazo[4,5-b]pyridin-2-yl)oxy)hexahydrofuro[3,2-*b*]furan-3-ol also known as MK-8722, effective in insulin-independent glucose uptake and glycogen synthesis, with resultant improvements in glycemia. However, the use of the compound is halted by the induction of cardiac hypertrophy caused by MK-8722. The clinical use of AMPK activators is thus, to this date, hampered by adverse effects of the compounds on metabolic pathways.

Thus, there exist a growing need for novel treatment regimens suitable in the treatment of metabolic diseases with greater efficacy, high safety (low toxicological effect), which also offers convenient and safe administration options.

### Summary of the invention

In view of the above, it is therefore an object of the present invention to provide an effective and safe therapeutic agent useful in the treatment of metabolic diseases, including obesity and NASH, in subjects in need thereof.

Accordingly, a first aspect of the present invention relates to a conjugated molecule comprising a peptide displaying at least 0.1 % activity of native glucagon at the glucagon receptor, and an AMP-activated protein kinase (AMPK) activator, wherein the peptide is covalently bonded to the AMPK activator either directly or through a linker.

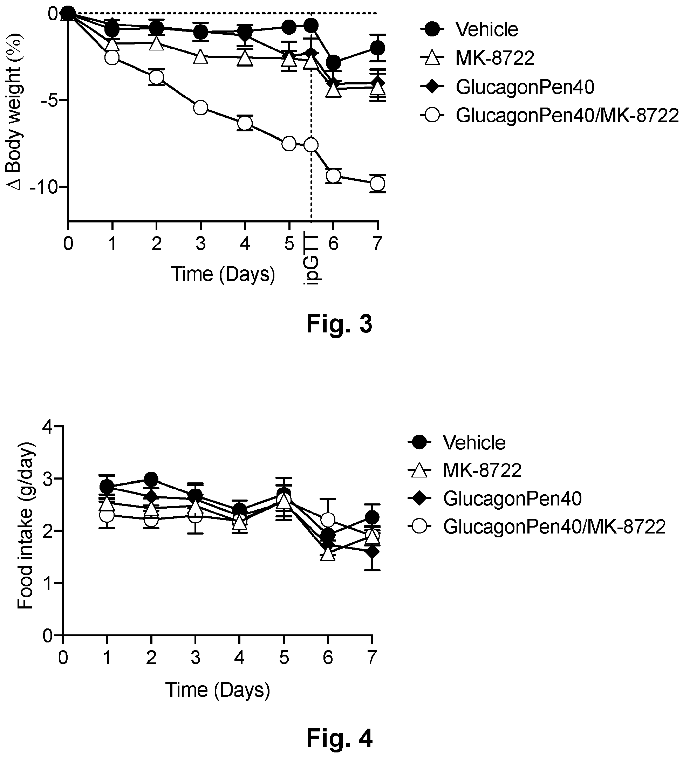

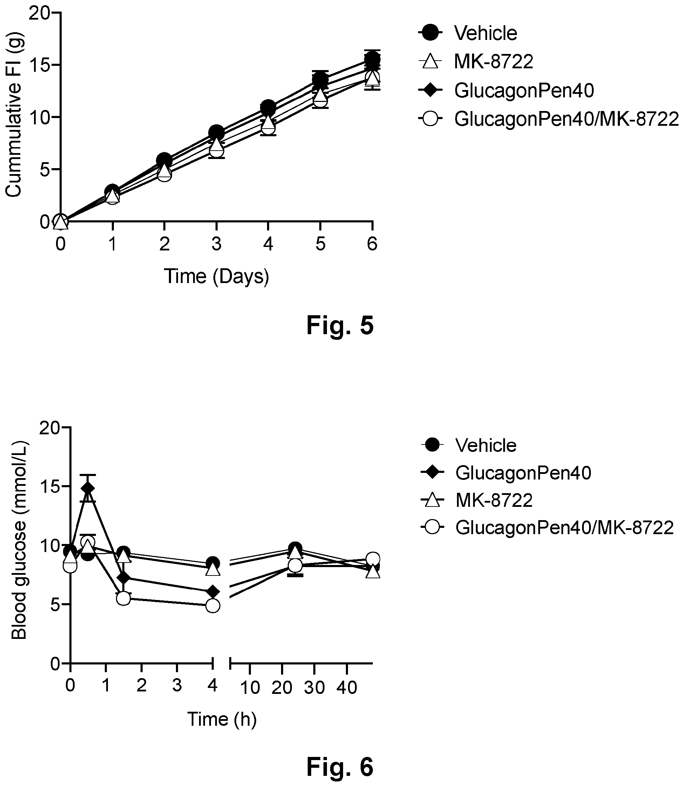

The inventors have surprisingly found that conjugation of peptides with glucagon agonism and AMPK activation represents a novel medicinal strategy for effectively reversing obesity, lowering plasma cholesterol and blood glucose levels in obese subjects. Conjugates based on this strategy are superior in causing a reduction body weight relative to the glucagon or MK-8722 alone, as shown in Fig. 3. Furthermore, the significant reduction in body weight achieved by the conjugates of the present inventive is independent of food intake, as shown in Figs. 4 and 5. Also, the conjugates are effective in lowering blood glucose and cholesterol levels, as shown in Figs. 6-8. The inventors have found that the ability of AMPK to improve glucose homeostasis is effective in cancelling the diabetic liabilities of glucagon, while the specific targeting achieved by glucagon restricts the activation of AMPK in cardiac tissue. Consequently, the conjugates of the present invention harvest the beneficial pharmacological effects on lipid metabolism from each of the individual components, while circumventing hallmark adverse effects. Without being bound by any particular theory, the inventors speculate that this effect is achieved by the peptide hormone which serves a bifunctional role as a lipolysis stimulating drug and as a targeting agent, allowing for site-selective delivery of the otherwise non-specific AMPK activator to the liver. Furthermore, the developed conjugates have a dual functionality, in that they combine AMPK activators with glucagon agonists, and is capable of targeting two distinct pathways involved in hepatic fatty acid oxidation.

A peptide will have an amino terminus and a carboxyl terminus. In the context of the invention, the amino terminus and the carboxyl terminus may also be referred to as the N-terminus and the C-terminus, respectively, and corresponding derived forms.

The peptide may consist of amino acids encoded by the genetic code or it may contain amino acids encoded by the genetic code and natural amino acids, which are not encoded by the genetic code, such as hydroxyproline, γ-carboxyglutamate, ornithine, phosphoserine, D-alanine (dAla), and D-glutamine. Further, the peptide may incorporate synthetic amino acids such as D-alanine, and D-leucine, or a-aminoisobutyric acid (Aib), d-Serine (dSer), N-methyl-serine.

The peptide may be obtained from any source or the peptide may be produced as desired. For example, the peptide may be isolated from a tissue, or the peptide may be produced recombinantly or synthesized by methods that are well known to the person skilled in the art.

The conjugated molecule comprises a peptide, the peptide (in its free form) displaying at least 0.1% activity of native glucagon at the glucagon receptor. In the context of the present invention, glucagon receptor activity, which may also be referred to as glucagon activation, can be measured in an *in vitro* assay by measuring cAMP induction in HEK293 cells over-expressing the glucagon receptor (GCCR). The assay may be carried out as described in, for example, Finan et al., Cell 167.3: 843-857 (2016), which is incorporated herein by reference. In an embodiment, the peptide of the conjugate displays at least 1% activity of native glucagon, such as at least 5%, 10%, 15%, 20%, or 30% activity.

The peptide of the conjugated molecule may be any peptide having at least 0.1% activity of native glucagon at the glucagon receptor. In an embodiment, the peptide of the conjugate is of the glucagon-superfamily. The glucagon-superfamily is a group of peptides related in structure in their N-terminal and C-terminal regions (see, for example, Sherwood et al., Endocrine Reviews 21: 619-670 (2000), which is incorporated herein by reference). Members of this group include glucagon related peptides, including, but not limited to, modified glucagon (SEQ ID NO: 1) and unmodified glucagon (SEQ ID NO: 2), and analogues, derivatives or conjugates with up to 1, 2, 3, 4, 5, 6, 7, 8, 9 or 10 amino acid modifications relative to the native peptide. Such peptides preferably retain the ability to interact with receptors of the glucagon receptor superfamily, preferably the glucagon receptor.

In an embodiment, the peptide of the conjugate has at least 80% amino acid sequence identity to SEQ ID NO:1. For example, the peptide may have at least about 80%, at least about 85%, at least about 90%, at least about 95%, or more than about 97% identity to SEQ ID NO:1. In one embodiment, the peptide has an amino acid sequence with at least 80% identity to SEQ ID NO:1. In another embodiment, the peptide has an amino acid sequence with at least 95% identity to SEQ ID NO:1. In a specific embodiment, the peptide has the amino acid sequence of SEQ ID NO:1. Such a peptide may have a significantly greater glucagon activity at the glucagon receptor compared to native glucagon at the glucagon receptor. As such, the peptide may, if conjugated to an AMPK activator, accumulate at a greater rate at the site of glucagon receptors, which in turn may lead to a greater efficacy of the AMPK activator.

The peptide of the conjugated molecule will have a length sufficient for the peptide (in its free form), to display at least 0.1 % activity of native glucagon at the glucagon receptor. In general, this can be observed for peptides comprising at least 10 amino acids, but the activity may not be displayed when the peptide comprises more than 60 amino acids. Thus, in an embodiment, the peptide has a length in the range of 10 to 60 amino acids, e.g. 20 to 50 amino acids. Amino acid sequences of the present invention that are identical to other peptides sequences to a certain percentage should comprise enough of the amino acid sequence of a peptide, e.g. at least 10 amino acids, to afford putative identification of that peptide, either by manual evaluation of the sequence by one skilled in the art, or by computer-automated sequence comparison and identification using algorithms such as BLAST (Basic Local Alignment Search Tool) (for a review see Altschul, et al., Meth Enzymol. 266: 460,1996; and Altschul, et al., Nature Genet. 6: 119, 1994).

In the context of the present invention, peptide may differ in %-identity by having substitutions, insertions of natural or synthetic amino acids and/or having amino acid deletions.

In an embodiment, the peptide is modified by acetylation, fatty acid conjugation, diacid conjugation, albumin conjugation, small-molecule albumin binders, and/or PEG conjugation. Also contemplated are peptides modified by linking to carrier proteins, such as antibodies. The modifications are preferably at position 16, 17, 20, 21, 24, and 29-40 of the peptide in SEQ ID NO:1 (counted from the N-terminal), within a C-terminal region, or at the C-terminal amino acid. Since none of the amino acids in positions 29 to 40 of SEQ ID NO:1 are directly involved in binding of peptide to the glucagon receptor, these amino acids may be exchanged and modified without influencing the binding of the peptide to the glucagon receptor. The conjugation may be made by any suitable linker, such as by disulfide, maleimide, alpha-ketone, or click-chemistry based conjugation. The skilled person knows how to prepare such conjugates. Preferably, PEG molecules may be larger than 1 kDa and fatty acids and diacids may contain more than 12 carbon atoms. It is generally preferred to add a spacer between the modification (PEG/fatty acid/diacid) and the peptide, the linker preferably being a gamma-Glu linker, a short PEG chain.

The conjugated molecule comprises an AMPK activator. Any AMPK activator may be used with the conjugate. However, it is preferred that the AMPK activator is a small molecule, e.g up to 900 kDa. For example, in one embodiment, the AMPK activator is selected from 5-Aminoimidazole-4-carboxamide 1-β-D-ribofuranoside (AICAR), (3R,3aR,6R,6aR)-6-((6-([1,1'-biphenyl]-4-yl)-7-chloro-3*H*-imidazo[4,5-b]pyridin-2-yl)oxy)hexahydrofuro[3,2-*b*]furan-3-ol (MK-8722), (3*R*,3a*R*,6*R*,6a*R*)-6-((6-chloro-5-(4-(1-(hydroxymethyl)cyclopropyl)phenyl)-1*H*-benzo[*d*]imidazol-2-yl)oxy)hexahydrofuro[3,2-*b*]furan-3-ol (PF-739), 6-Chloro-5-[4-(1-hydroxycyclobutyl)phenyl]-1H-indole-3-carboxylic acid (PF-06409577), 1,1-Dimethylbiguanide hydrochloride (metformin), 4-Hydroxy-3-(2'-hydroxy-1,1'-biphenyl-4-yl)-6-oxo-6,7-dihydrothieno[2,3-b]pyridine-5-carbonitrile (A-769662), 2-Chloro-5-[[5-[[5-(4,5-Dimethyl-2-nitrophenyl)-2-furanyl]methylene]-4,5-dihydro-4-oxo-2-thiazolyl]amino]benzoic acid (PT-1), 2-[[2-(2-Bromo-4-methylphenoxy)ethyl]thio]-pyrimidine (ZLN024), 2-[[4-(Diethyl-amino)-2-hydroxyphenyl]methylene]hydrazide-4-pyridinecarboxylic acid (RSVA-405) , and analogues thereof. MK-8722, PF-739 and PF-06409577 are preferred.

The peptide of the invention and the AMPK activator are covalently bonded. In the context of the present invention, the conjugated molecule may also be referred to as a peptide-drug-conjugate (PDC). The peptide and the AMPK activator may be bonded directly to each other. For example, the AMPK activator may be bonded covalently through an ether bond, an ester bond, a carbamate bond, a carbonate bond, a triazole bond, a maleimide bond and an amide bond.

In the context of the present invention, being directly covalently bonded means that the peptide has a covalent bond with the AMPK activator, e.g. there are no additional chemical groups between the two molecules, such as a linker group.

In one embodiment, the AMPK activator is covalently bonded at the C-terminal region of the peptide. In the context of the invention, the C-terminal region may be up to 50% of the amino acids counted from the C-terminus, such as up to 40%, 30%, 25%, 20%, or 10% of the amino acids counted from the C-terminus. For instance, the C-terminal region of SEQ ID NO:1 may be amino acids 15 to 40, 26 to 40, or 30 to 40 (numbers counted from N-terminal). Thus, the AMPK activator may be bonded, either directly or via a linker, to any one of the 10 amino acids counted from the C-terminus. Thereby the AMPK activator produces little or no steric hindrance at the N-terminal of the peptide. It is also contemplated that more than one AMPK activator may be bonded to the same peptide molecule.

The peptide and the AMPK activator may be bonded through a linker. Any linker may be used. However, it is generally preferred that the linker has a length of up to 30 atoms. A longer chain may have the advantage of distancing the AMPK activator from the peptide, such that the AMPK activator is of no or little steric hindrance to the peptide, when the peptide interacts with a glucagon receptor, and vice versa. No or low steric hindrance of the peptide affords a greater affinity towards the glucagon receptor. A conjugate with a greater affinity towards the glucagon receptor is likely to have a greater accumulation at the site of glucagon receptors.

In one embodiment the AMPK activator is covalently bonded to the peptide via a cleavable chemical linker, the cleavable chemical linker being selected from acid-cleavable linkers, enzyme-cleavable linkers, peptide-cleavable linkers, and linkers comprising a disulfide group. Such linkers are generally well-known in the art for their use in peptide-drug conjugates. Examples of such cleavable linkers are compounds comprising glucuronide, beta-galactoside, disulfide, hydrazone and/or which compounds are cleavable by galactosidases, glucuronidases, pyrophospatases, phosphatases, arylsulfatases, proteases, or esterases. The linkers preferably release the AMPK activator in its free form (i.e. native form), which may be achieved by many different linker chemistries such as the disulfide linkers disclosed herein. These linker chemistries and additional linker chemistries are well-known by the skilled person.

In a preferred embodiment, the AMPK activator is covalently bonded to the peptide via a chemical linker comprising a disulfide group. A disulfide group allows for the release of AMPK activator from the peptide when chemically reduced. A chemical linker comprising a disulfide group, also known as a disulfide linker, ensures that the peptide and the AMPK activator of the conjugate remain conjugated for an extended period during systemic circulation. The disulfide group of the disulfide linker may be reduced in a reducing environment, such as an intracellular environment, resulting in the conjugate being cleaved such that the peptide part of the conjugate is separated from the AMPK activator part of the conjugate. The reduction may be through disulfide exchange with e.g. a thiol, such as glutathione or reductases such as intracellular protein disulfide-isomerase enzymes.

The chemical linker may be chosen from chemical linkers known in the art with the general formula R'-S-S-R", in which the R' and R" groups may be identical or different from each other. Advantageously, the conjugate may accumulate at and/or close to the sites of glucagon receptors in the body due to the affinity of the peptide towards glucagon receptors, and the AMPK activator may be released at the sites and/or close to the sites of the glucagon receptors. When free from peptide part of the conjugate, the AMPK activator may have a suitably effect as site-specific AMPK activator. It is speculated by the inventors that the conjugate may be cleaved in the extracellular environment immediately adjacent to cells harbouring glucagon receptors, or that the conjugate may be internalized by the cells harbouring glucagon receptors and cleaved in the reducing environment of the cells.

In one embodiment, the conjugated molecule is conjugated via a chemical linker, wherein the chemical linker has the formula R₁-R₃-S-S-R₄-R₅-O-CO-R₂, wherein R₁ is the peptide, R₂ the AMPK activator, R₃ is optional and when present is selected from C(CH₃)₂, CH₂-CH₂, or CH₂, bonded to a side chain of the peptide or to a carbon atom of the backbone chain of the peptide, R₄ is (CH₂)ₙ or C₆H₄, R₅ is optional and when present is selected from C(CH₃)₂, CH-CH₃, CH₂-CH₂, or CH₂, and n is 1, 2, or 3. When the chemical linker is reduced, the liberated AMPK activator part of the conjugate undergoes intramolecular cyclisation which leads to the release of the AMPK activator into its free form.

In one embodiment, the chemical linker has the formula R₁-R₃-S-S-(CH₂)ₙ-NH-CO-R₂, wherein R₁ is the peptide, R₂ the AMPK activator, R₃ is optional and when present is selected from C(CH₃)₂, CH₂-CH₂, or CH₂, bonded to a side chain of the peptide or to a carbon atom of the backbone chain of the peptide, and n is 1, 2, or 3.

In one embodiment, the chemical linker has the formula R₁-R₄-R₃-S-S-(CH₂)ₙ-NH-CO-R₂, wherein R₁ is the peptide, R₂ the AMPK activator, R₃ is optional and when present is selected from CH(CH₃)₂, CH₂-CH₂, or CH₂, bonded to a side chain of the peptide or to a carbon atom of the backbone chain of the peptide, R₄ is optional and when present is selected from CH(CH₃)₂, CH₂-CH₂, or CH₂, bonded to a side chain of the peptide or to a carbon atom of the backbone chain of the peptide and n is 1, 2, or 3.

In an embodiment, the second radical bond is to the backbone of the peptide of the invention.

In another embodiment, the second radical bond is to a side chain of the peptide of the invention.

In the context of the present invention, when R₁ is bonded to the backbone chain of the peptide, C(CH₃)₂ (L-penicillamine) may be referred to as Pen, CH₂-CH₂ (L-homocysteine) may be referred to as hCys, and CH₂ (L-Cysteine) may be referred to as Cys.

As used herein, the first and the second radical bond is used to state the presence of at least two free bonds in the chemical linkers disclosed herein.

In another embodiment, the AMPK activator is covalently bonded to the peptide via a non-cleavable linker, wherein the non-cleavable linker is selected from polyethylene glycol (PEG) linkers, carbon linkers, SMCC and mc with conjugation chemistries of maleimides, ethers, amides, triazoles, disulfide, and thioether.

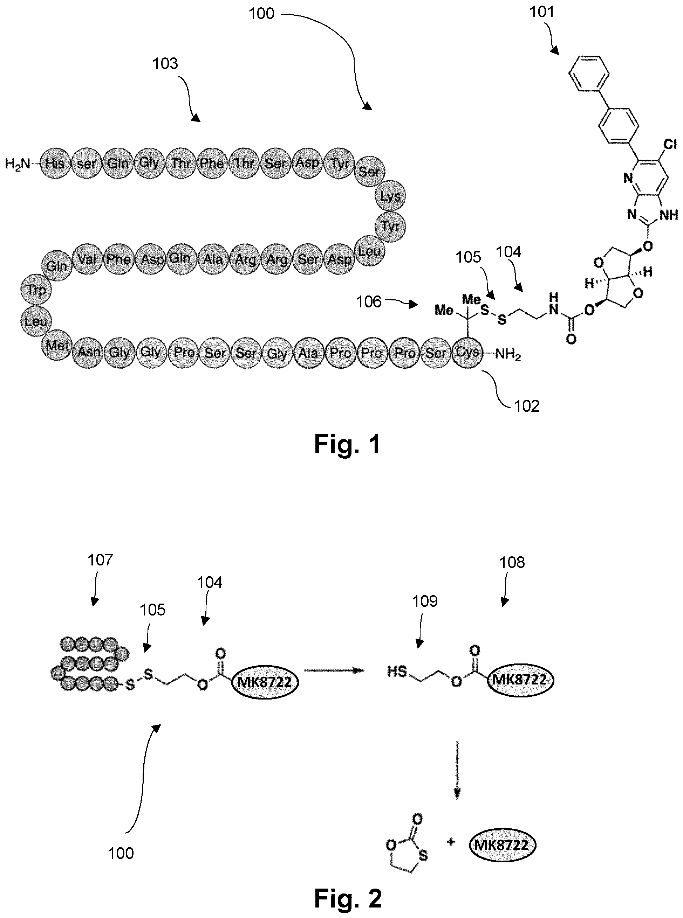

The present invention facilitates the design and synthesis of a library of conjugated molecules comprising a peptide and an AMPK activator appended via chemical linkers. Fig. 1 shows how such conjugated molecules, may be designed. As shown in Fig. 1, the conjugate may be prepared by chemically bonding an AMPK activator (MK-8722 in Fig. 1) to a peptide. The skilled person will appreciate that a vast number of different chemical linkers may be prepared by the methods disclosed herein and by other methods reported in literature, and these chemical linkers may be used to append peptides and AMPK activator to the methods disclosed herein and as reported elsewhere in the known art.

The inventors have surprisingly found that the peptide of the present invention may serve a bifunctional role as a weight lowering drug and a targeting agent, allowing for site-selective delivery of otherwise non-specific small-molecules, such as AMPK activators, to the liver. It is understood, that the targeting properties of the peptide of the present invention may also facilitate delivery of the AMPK activators to other sites, such as for example the endocrine pancreas.

The conjugated molecule disclosed herein provides selectivity and also up-concentrates drug action in the targeted region. This targeting enabled by the conjugated molecule allows for an improved therapeutic index, i.e. a lower minimum effective concentration. Furthermore, the coupling allows to add-on another layer of metabolic drug action to the efficacy of glucagon receptor targeting medicines..

The inventors have demonstrated a surprising synergistic effect of the conjugates of the invention on reduction on body weight and this is significantly greater in comparison to the effect obtained with the administration of peptide or the drug alone, see Fig. 3. Interestingly, the food intake shows that the weight loss is uncoupled from appetite suppression, suggesting its effect is purely driven by increased energy expenditure, see Figs. 4 and 5.

The inventors have further demonstrated a surprising synergistic effect of the conjugates of the invention on lowering blood glucose and plasma cholesterol, and this is significantly greater in comparison to the effect obtained with the administration of the peptide or the drug alone, see Figs. 6 to 8.

In an embodiment of the present invention the conjugated molecule is for use in therapy.

In an embodiment, the conjugated molecule of the present invention is for use in the treatment of obesity, type 2 diabetes, hyperinsulinemia, insulin resistance, impaired glucose tolerance, hypercholesterolaemia, non-alcoholic fatty liver disease (NAFLD), non-alcoholic steatohepatitis (NASH) and dyslipidemiae.

Another aspect of the present invention relates to a pharmaceutical composition comprising the conjugated molecule according to the invention, and a pharmaceutically acceptables carrier. Any embodiment of the conjugated molecule may be used in the pharmaceutical composition.

The invention relates to the use of the conjugated molecule according to the invention in the manufacture of a pharmaceutical composition. In particular, the pharmaceutical composition is for use in the treatment of obesity, type 2 diabetes, hyperinsulinemia, insulin resistance, impaired glucose tolerance, hypercholesterolemia, non-alcoholic fatty liver disease (NAFLD), non-alcoholic steatohepatitis (NASH) and dyslipidemia. Any embodiment of the conjugated molecule may be used in the manufacture of the pharmaceutical composition.

The data disclosed in the present invention have been obtained in studies of mice, but the conclusions are equally relevant for humans, since the major hormonal pathways governing energy metabolism are similar between mice and humans at they display comparable receptor expression profiles.

The conjugate of the present invention may be administered in the form of a pharmaceutical composition. Accordingly, the present invention further provides a pharmaceutical composition, which comprises a conjugate of the present invention or a pharmaceutically acceptable salt thereof, and a pharmaceutically acceptable carrier. The pharmaceutical formulations may be prepared by conventional techniques. Briefly, pharmaceutically acceptable carriers can be either solid or liquid. Solid form preparations include powders, tablets, pills, capsules, cachets, suppositories, and dispersible granules. A solid carrier can be one or more excipients which may also act as diluents, solubilizers, lubricants, suspending agents, binders, preservatives, wetting agents, tablet disintegrating agents or an encapsulating material.

The conjugate comprised in the pharmaceutical formulation may be in powder form, obtained by aseptic isolation of sterile solid or by lyophilisation from solution for constitution before use with a suitable vehicle, e.g., sterile, pyrogen-free water.

In one embodiment, the pharmaceutical composition is suited for subcutaneous administration, intramuscular administration, intraperitoneal administration, intravenous administration or for oral administration. Accordingly, the compositions of the present invention may be provided in unit dose form in ampoules, pre-filled syringes, small volume infusion or in multi-dose containers, optionally with an added preservatives. The compositions may take such forms as suspensions, solutions or emulsions in oily or aqueous vehicles.

In accordance with the current disclosure, pharmaceutical compositions are provided wherein the weight lowering effects of peptides with glucagon activity are combined with AMPK activator in a single modality. Active delivery via peptides with glucagon activity to the liver avoid unwanted adverse effects, such as for examples cardiac hypertrophy caused by AMPK activation. Positive metabolic effects of AMPK activators may include improvements in glucose uptake, fatty acid oxidation, mitochondrial biogenesis and insulin sensitivity, which may be beneficial for reducing obesity and obesity-related metabolic disorders in humans or mammal.

Thus, the therapeutic utility of a peptide of the invention and AMPK activator pairing offers a new approach for the treatment of obesity and its associated metabolic disorders.

A further aspect of the present invention relates to a method of reducing body weight in a mammal comprising administering the conjugated molecule of the invention or the pharmaceutical composition of the invention.

The conjugated molecule or the pharmaceutical composition may be administered subcutaneously, orally, intramuscularly, intraperitoneally, or intravenously.

The conjugated molecule, and thus also the pharmaceutical composition, is effective in reducing body weight. Therefore, the conjugated molecule and the pharmaceutical composition may be used in the treatment of obesity at any level. Obesity may be described in terms of the body mass index (BMI), which is deffined as the body mass divided by the square of the body height, e.g. as expressed in units of kg/m². Without being bound by theory, the present inventors consider that the BMI can be used to define a limit between pathogenic obesity and non-pathogenic obesity. For example, in the context of the invention, a BMI of 30 kg/m² may be interpreted as the limit between pathogenic obesity and non-pathogenic obesity. However, other values of BMI can also be considered to define the limit between pathogenic obesity and non-pathogenics obesity. Thus, for example, BMI values of 24 kg/m², 25 kg/m², 26 kg/m², 27 kg/m², 28 kg/m², 29 kg/m², 30 kg/m², 31 kg/m², 32 kg/m², 33 kg/m², 34 kg/m², and 35 kg/m² are considered to define the limit between pathogenic obesity and non-pathogenic obesity.

In a further aspect, the present invention relates to a non-therapeutic treatment of mammals for reducing body weight comprising orally administering to said mammal the conjugated molecule according to the invention. For example, the mammal may have a non-pathogenic BMI. In particular, the method may comprise orally administering the conjugated molecule to a subject having a BMI below the limit defining non-pathogenic obesity.

In the above, the invention has mainly been described with reference to a few embodiments. However, as is readily appreciated by a person skilled in the art, other embodiments than the ones disclosed above are equally possible within the scope of the invention.

Other aspects and advantageous features of the present invention are described in detail and illustrated by non-limiting working examples below.

Generally, all terms used herein are to be interpreted according to their ordinary meaning in the technical field, and applicable to all aspects and embodiments of the invention, unless explicitly defined or stated otherwise. All references to "a/an/the [conjugate, molecule, linker, peptide, etc.]" are to be interpreted openly as referring to at least one instance of said conjugate, agent, molecule, linker, peptide, etc., unless explicitly stated otherwise.

In the context of the present invention, the term glucagon means a peptide of the glucagon-superfamily. The peptide of the invention may also be considered to be weight regulating hormone peptides and to function as an active delivery agent of the conjugated molecule of the present invention to the liver.

In the context of the present invention, the term "peptide" means a compound composed of stretch of 10 to 60 amino acids connected by peptide bonds.

The term "analogue" as used herein in relation to glucagon peptide and AMPK activators means a peptide or compound or drug having an analogous or similar biological property and effect.

The term "derivative" as used herein in relation to a peptide or an amino acid means a chemically modified peptide or amino acid, wherein at least one substituent is not present in the unmodified peptide or amino acid or analogues thereof, i.e. a peptide or an amino acid which has been covalently modified. Typical modifications are amides, carbohydrates, alkyl groups, acyl groups, esters and the like.

In the context of the present invention, the term "percentage identity" or "% identity" means % of identical amino acids between two compared peptides, in particular using the BLAST algorithm.

The term "AMPK activator" as used herein means a compound which is an activator or agonist or stimulant of AMP-activated protein kinase (AMPK). Examples of AMPK activators include, but are not limited to, 5-Aminoimidazole-4-carboxamide 1-β-D-ribofuranoside (AICAR), (3R,3aR,6R,6aR)-6-((6-([1,1'-biphenyl]-4-yl)-7-chloro-3*H*-imidazo[4,5-b]pyridin-2-yl)oxy)hexahydrofuro[3,2-*b*]furan-3-ol (MK-8722), (3*R*,3a*R*,6*R*,6a*R*)-6-((6-chloro-5-(4-(1-(hydroxymethyl)cyclopropyl)phenyl)-1*H*-benzo[*d*]imidazol-2-yl)oxy)hexahydrofuro[3,2-*b*]furan-3-ol (PF-739), 6-Chloro-5-[4-(1-hydroxycyclobutyl)phenyl]-1H-indole-3-carboxylic acid (PF-06409577), 1,1-Dimethylbiguanide hydrochloride (metformin), 4-Hydroxy-3-(2'-hydroxybiphenyl-4-yl)-6-oxo-6,7-dihydrothieno[2,3-b]pyridine-5-carbonitrile (A-769662), 2-Chloro-5-[[5-[[5-(4,5-Dimethyl-2-nitrophenyl)-2-furanyl]methylene]-4,5-dihydro-4-oxo-2-thiazolyl]amino]benzoic acid (PT-1), 2-[[2-(2-Bromo-4-methylphenoxy)ethyl]thio]-pyrimidine (ZLN024), 2-[[4-(Diethylamino)-2-hydroxyphenyl]methylene]hydrazide-4-pyridinecarboxylic acid (RSVA-405).

### Brief description of figures

The above, as well as additional objects, features, and advantages of the present invention is better understood through the following illustrative and non-limiting detailed description of embodiments of the present invention, with reference to the appended drawings, wherein:
Fig. 1 shows an example of a peptide and AMPK activator conjugate,
Fig. 2 displays the mechanism by which MK-8722 is released from the conjugate of Fig. 1,
Fig. 3 shows the weight-lowering effect of a conjugate of a peptide of SEQ ID NO:1 and MK-8722 (Glucagon Pen40/MK-8722),
Fig. 4 shows the effect of Glucagon Pen40/MK-8722 conjugate on daily food intake in mice,
Fig. 5 shows the effect of Glucagon Pen40/MK-8722 conjugate on cumulative food intake in mice,
Fig. 6 shows the effect of Glucagon Pen40/MK-8722 conjugate on blood glucose in mice after a compound tolerance test (CTT),

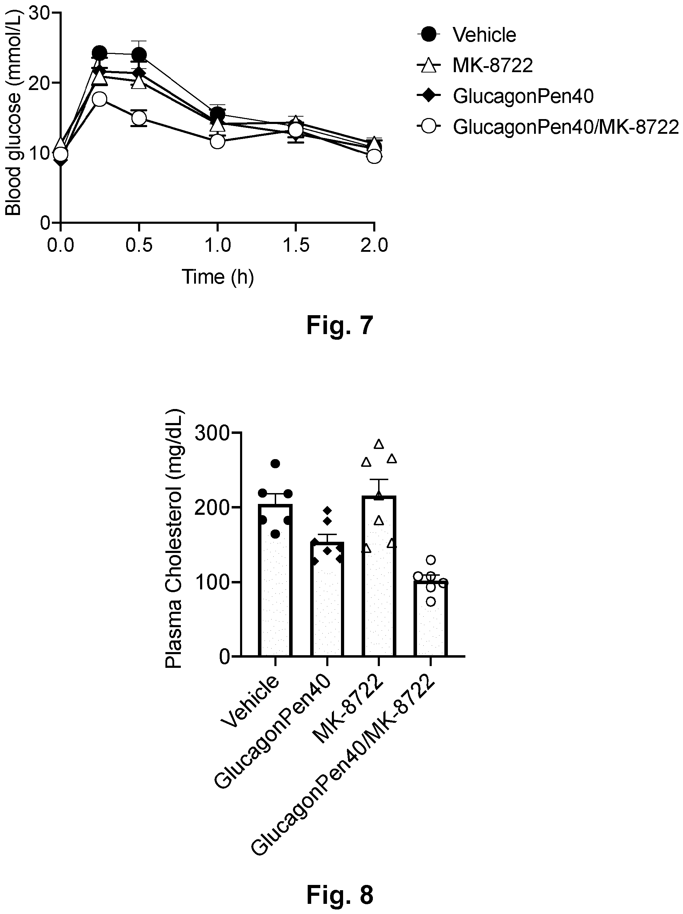

Fig. 7 shows the effect of Glucagon Pen40/MK-8722 conjugate on blood glucose in mice after an intraperitoneal glucose tolerance test (IpGTT),
Fig. 8 shows the effect of Glucagon Pen40/MK-8722 conjugate on plasma cholesterol in mice,

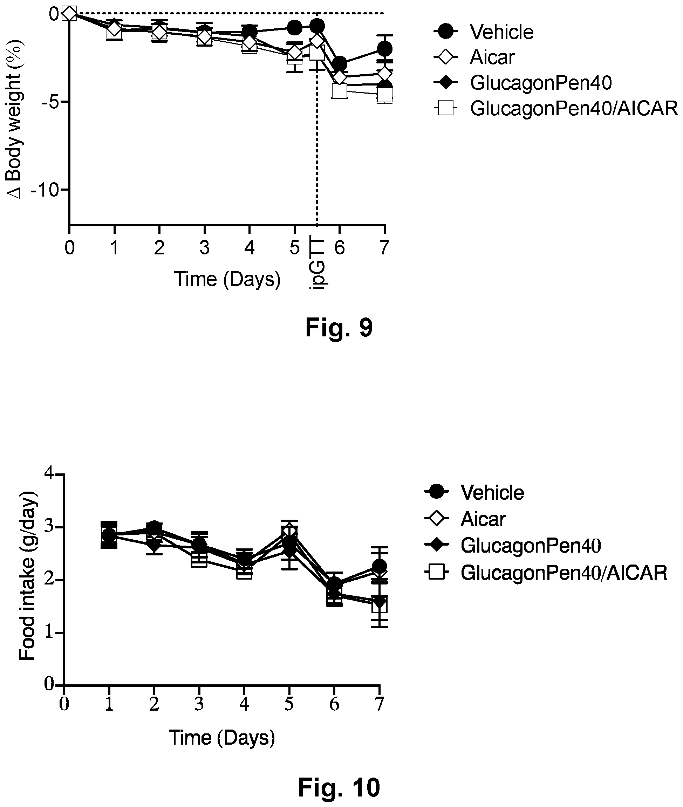

Fig. 9 shows the weight-lowering effect of a conjugate of a peptide of SEQ ID NO:1 and AICAR (Glucagon Pen40/AICAR),
Fig. 10 shows the effect of Glucagon Pen40/AICAR conjugate on daily food intake in mice,

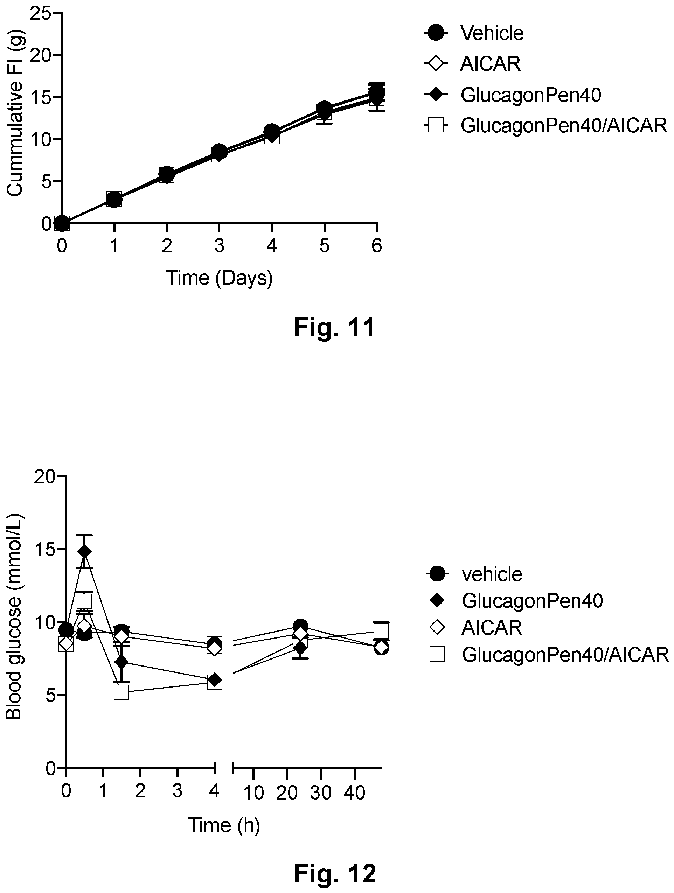

Fig. 11 shows the effect of Glucagon Pen40/AICAR conjugate on cumulative food intake in mice,
Fig. 12 shows the effect of Glucagon Pen40/AICAR conjugate on blood glucose in mice after a compound tolerance test (CTT),

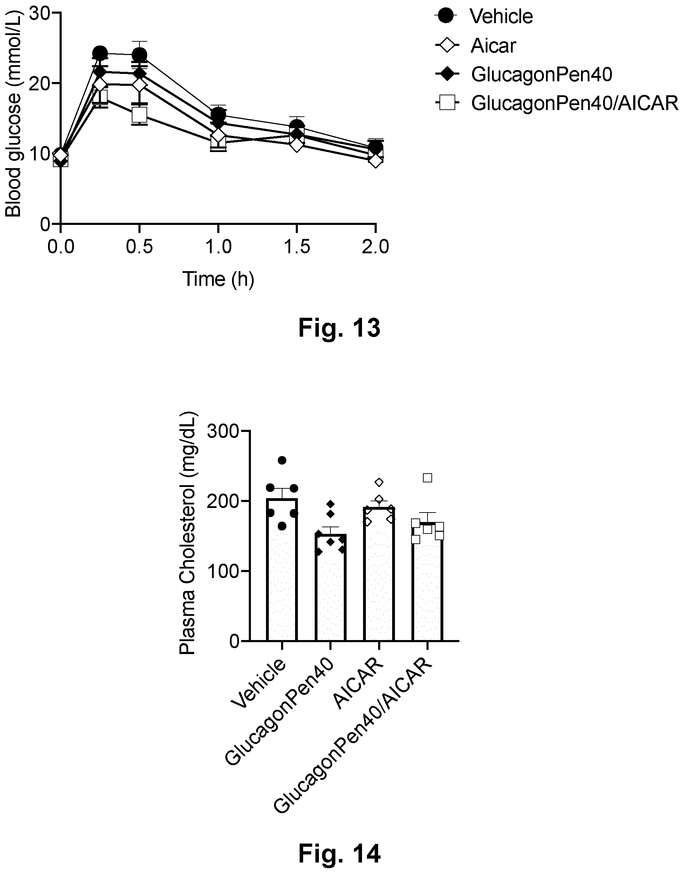

Fig. 13 shows the effect of Glucagon Pen40/AICAR conjugate on blood glucose in mice after an intraperitoneal glucose tolerance test (IpGTT),
Fig. 14 shows the effect of Glucagon Pen40/AICAR conjugate on plasma cholesterol in mice,

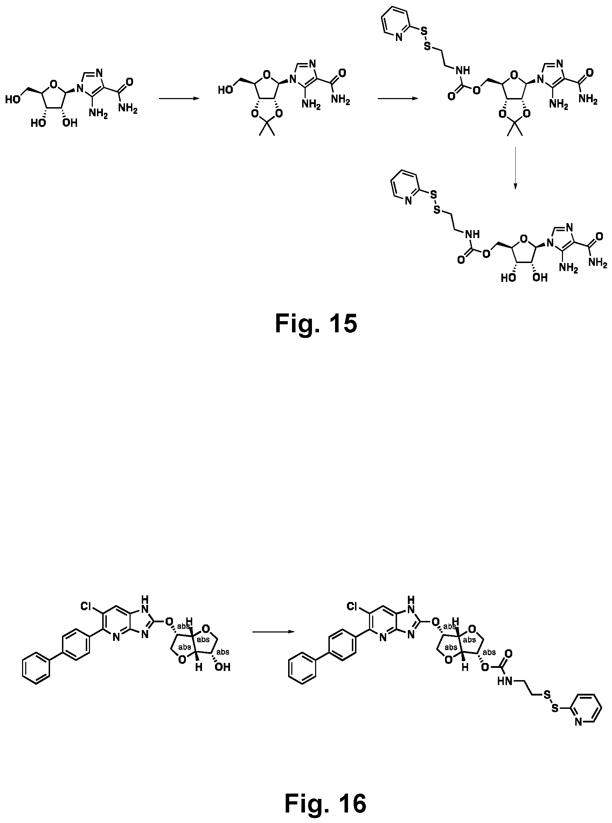

Fig. 15 shows a synthesis route of a chemical linker derivatized AICAR, and
Fig. 16 shows a synthesis route for chemical linker derivatized MK-8722.

### Detailed description

Fig. 1 shows an example of a peptide and AMPK activator conjugate 100, which consists of MK-8722 101 chemically appended to a C-terminal cysteine 102 of the peptide of SEQ ID NO:1 103 through a chemical linker 104, the chemical linker 104 comprising a disulfide group 105. A side chain 106 of the C-terminal cysteine 102, may optionally be derivatised, such that length n of the side chain 106 is 1 or 2 carbon atoms and/or R is hydrogen or methyl. A modification called hCys40 of the side chain 106 has length n = 2 carbon atom and R = hydrogen. A modification called hCys40 of the side chain 106 has length n = 1 carbon atom and R = methyl. Regular cysteine is called Cys40.

Fig. 2 displays the mechanism by which MK-8722 is released from the conjugate 100 of Fig. 1. The chemical linker 104 comprising a disulfide group 105 is self-immolative and may be reduced in a reducing environment (not shown) such as an intracellular environment to produce thiol groups, separating the peptide part of the conjugate 107 from the MK-8722 part 108 of the conjugate. On the MK-8722 part 108 of the molecule, a liberated nucleophilic thiol 109 undergoes spontaneous intramolecular cyclization to release MK-8722 as the native unmodified MK-8722 drug (free form of MK-8722).

Fig. 3-14 display the results of the in vivo mice studies disclosed in Example 2.

Fig. 3 shows the weight-lowering effect of a conjugate of a peptide of SEQ ID NO:1 and MK-8722 chemically appended via the linker shown in Fig. 1 and 2 (Glucagon-Pen40/MK-8722) (100nmol/kg) and equimolar doses of the peptide of SEQ ID NO:1 (Glucagon Pen40) or MK-8722 measured in body weight percentage (BW %) of diet-induced obesity (DIO) mice treated for 7 days. Data is expressed as mean ± SEM and N is 8 per group. Mice treated with monotherapies of either Glucagon Pen40 or MK-8722 showed a slight reduction in body weight, appr. 4 % BW % reduction. The highest reduction in BW % was observed in DIO mice treated with Glucagon Pen40/MK-8722, the conjugate giving rise to approximately 10 % BW % reduction after 7 days of treatment. Additionally, based on the slope of the curve, it would seem that a further reduction in body weight could be expected for the Glucagon Pen40/MK-8722 if treatment was extended.

Fig. 4 shows the effect of Glucagon Pen40/MK-8722 (100nmol/kg) or equimolar doses of Glucagon Pen40 or MK-8722 on daily food intake (gram per day) in DIO mice treated for 7 days. Data is expressed as mean ± SEM and N = 8 per group. During the 7 days of treatment, mice treated with Glucagon Pen40, MK-8722 or Glucagon Pen40/MK-8722 showed similar daily food intake as compared to the control group of mice (Vehicle, i.e. saline). At the end of the study, mice treated with Glucagon Pen40 showed a slight reduction in food intake compared to the control group (vehicle).

Fig. 5 shows the effect of Glucagon Pen40/MK-8722 and equimolar doses of Glucagon Pen40 or MK-8722 on cumulative food intake (Cumulative FI, gram per day) in DIO mice treated for 6 days. Data is expressed as mean ± SEM and N = 8 per group. Over the course of the treatment, mice treated with Glucagon Pen40, MK-8722 or Glucagon Pen40/MK-8722 showed similar cumulative food intake as compared to the control group of mice (Vehicle, i.e. saline).

Fig. 6 and 7 show the effect of Glucagon Pen40/MK-8722 and equimolar doses of Glucagon Pen40 or MK-8722 compared to a control group (vehicle, i.e. saline) on blood glucose level (mmol/L) in DIO mice subjected to either a compound tolerance test (CTT) (Fig. 6) or Intraperitoneal glucose tolerance test (ipGTT) (Fig. 7) on day 7 of the treatment course. The blood glucose levels were measured over a course of 40 hours (CTT, Fig. 6) and 2 hours (IpGTT, Fig. 7). Data is expressed as mean ± SEM, N = 5 to 6 per group. In general, Glucagon Pen40/MK872 results in a significantly lower initial increase in blood glucose levels (immediately after stressing, >1h) compared to the control group.

Fig. 8 shows the effect of Glucagon Pen40/MK-8722 and equimolar doses of Glucagon Pen40 or MK-8722 compared to a control group (vehicle, i.e. saline) on plasma cholesterol level (mg/dL) in DIO mice treated for 7 days. Data is expressed as mean ± SEM, N = 5 to 6 per group. A significant reduction in plasma cholesterol level was observed for mice treated with Glucagon Pen40/MK-8722. Furthermore, mice treated with the conjugate not only displayed a much lower plasma cholesterol level compared to the vehicle 100 mg/dL vs. 200 mg/dL), but the conjugate also clearly outperformed MK-8722 and Glucagon Pen40 administered as monotherapies.

Fig. 9 shows the weight-lowering effect of a conjugate of a peptide of SEQ ID NO:1 and AICAR chemically appended via the linker shown in Fig. 1 and 2 (Glucagon-Pen40/AICAR) (100nmol/kg) and equimolar doses of the peptide of SEQ ID NO:1 (Glucagon Pen40) or AICAR measured in body weight percentage (BW %) of diet induced (DIO) mice treated for 7 days. Data is expressed as mean ± SEM and N is 8 per group. Mice treated with monotherapies of either Glucagon Pen40 or AICAR or the conjugate Glucagon Pen40/AICAR showed only a slight reduction in body weight, appr. 4 % BW % reduction, compared to control group (Vehicle, i.e. saline). The highest reduction in BW % was observed in DIO mice treated with either Glucagon Pen40 alone or the conjugate Glucagon Pen40/AICAR, both giving rise to approximately 5 % BW % reduction after 7 days of treatment.

Fig. 10 shows the effect of Glucagon Pen40/AICAR (100nmol/kg) or equimolar doses of Glucagon Pen40 or AICAR on daily food intake (gram per day) in DIO mice treated for 7 days. Data is expressed as mean ± SEM and N = 8 per group. During the 7 days of treatment, mice treated with Glucagon Pen40, MK-8722 or Glucagon Pen40/MK-8722 showed similar daily food intake as compared to the control group of mice (Vehicle, i.e. saline). At the end of the study, mice treated with Glucagon Pen40 or Glucagon Pen40/AICAR showed a slight reduction in food intake compared to the control group (vehicle) and AICAR. Based on the slope of the curve, it would seem that a negative trend in food intake could be expected for the Glucagon Pen40/AICAR or Glucagon Pen40 if treatment was extended.

Fig. 11 shows the effect of Glucagon Pen40/AICAR and equimolar doses of Glucagon Pen40 or AICAR on cumulative food intake (Cumulative FI, gram per day) in DIO mice treated for 6 days. Data is expressed as mean ± SEM and N = 8 per group. Over the course of the treatment, mice treated with Glucagon Pen40, MK-8722 or Glucagon Pen40/MK-8722 showed similar cumulative food intake as compared to the control group of mice (Vehicle, i.e. saline).

Fig. 12 and 13 show the effect of Glucagon Pen40/AICAR and equimolar doses of Glucagon Pen40 or AICAR compared to a control group (vehicle, i.e. saline) on blood glucose level (mmol/L) in DIO mice subjected to either CTT (Fig. 12) or ipGTT (Fig. 13) on day 7 of the treatment course. The blood glucose levels were measured over a course of 40 hours (CTT, Fig. 12) and 2 hours (IpGTT, Fig. 13). Data is expressed as mean ± SEM, N = 5 to 6 per group. In both stress tests, Glucagon Pen40/AICAR results in a significantly lower initial increase in blood glucose levels (immediately after stressing, >1h) compared to the control group.

Fig. 14 shows the effect of Glucagon Pen40/AICAR and equimolar doses of Glucagon Pen40 or AICAR compared to a control group (vehicle, i.e. saline) on plasma cholesterol level (mg/dL) in DIO mice treated for 7 days. Data is expressed as mean ± SEM, N = 5 to 6 per group. The highest reduction in plasma cholesterol level was observed for mice treated with Glucagon Pen40 or Glucagon Pen40/MK-8722. Mice treated with AICAR alone showed similar plasma cholesterol levels as the control group, and it was concluded that the effect AICAR on plasma cholesterol is limited..

### Conclusion

The presented data demonstrate that chemical conjugation of a Glucagon analogue and an AMPK activator represents a novel medicinal strategy for effectively reversing metabolic disorders, including obesity, diabetes and NASH. Conjugates based on this strategy are superior in lowering body weight and cholesterol levels relative to the glucagon control and are not flawed with adverse central effects of AMPK activator.

### Examples

### Example 1: Preparation of peptides and peptide-AMPK activator conjugates.

**Materials:** All solvents and reagents were purchased from commercial sources and used without further purification. H-Rink amide ChemMatrix^{®} resin was used for peptide elongation. Unless otherwise stated Fmoc-protected (9-fluorenylmethyl carbamate) amino acids were purchased from Iris-Biotech or Gyros Protein Technologies, and H-Rink amide ChemMatrix^{®} resin, 35-100 mesh; loading of 0.40 - 0.60 mmol/g from Sigma Aldrich. The commercially available *N^{α}*-Fmoc amino acid building blocks were purchased as the following sidechain protected analogs: Arg, Pmc; Asp, O^{t}Bu; Cys, Trt; Gln, Trt; His, Trt; Lys, Trt; Ser, ^{t}Bu; and Trp, Boc (Pmc = 2,2,5,7,8-pentamethylchoman-6-sulfonyl, O^{t}Bu = *tert*-butyl ester, Trt = trityl, Boc = *tert*-butyloxycarbonyl, and ^{t}Bu = *tert*-butyl ether).

All peptides and conjugates of peptides and AMPK activators were characterized by analytical reverse phase ultra-performance liquid chromatography (RP-UPLC) (Waters) and electrospray ionization liquid chromatography mass spectrometry (ESI-LCMS) coupled to a Agilent 6410 Triple Quadrupole Massfilter with a C18 column (Zorbax Eclipse, XBD-C18, 4.6 × 50 mm). The ESI-LCMS was eluting with a binary buffer system composed of H₂O:MeCN:TFA (A: 95:5:0.1, B: 5:95:0.1) at a flow rate of 0.75 mL/min. Purities were determined by RP-UPLC equipped with a C18 column (Acquity UPLC BEH C18, 1.7 µm, 2.1 × 50 mm) eluting with a binary buffer system composed of H₂O:MeCN:TFA (A: 95:5:0.1, B: 5:95:0.1) at a flow rate of 0.45 mL/min.

**Automated peptide synthesis protocol for Fmoc-protection scheme:** Peptides were prepared as their C-terminally amidated derivatives using a Prelude X, induction heating assisted, peptide synthesizer (Gyros Protein Technologies, Tucson, AZ, USA) with 10 mL glass vessels. All reagents were freshly prepared as stock solutions in DMF: Fmoc-protected amino acid (0.2 M), HCTU (0.5 M), DIPEA (1.0 M) and piperidine (20 % v/v). Peptide elongation was achieved by consecutive synthetic manipulations using the following protocol: Deprotection (2 × 2 min, RT, 300 rpm shaking) and coupling (2 × 5 min, 75°C, 300 rpm shaking, for Arg and His 2 × 5 min, 50°C, 300 rpm shaking). Peptides were prepared using double and triple couplings consisting of AA/HCTU/DIPEA (ratio 1:1.25:2.5) in 5-fold excess compared to the resin.

**Purification:** The crude peptide or conjugates of peptides and AMPK activator was analyzed by RP-UPLC and ESI-LCMS or MALDI-TOF mass spectrometry prior to purification. Purifications were performed with a reverse-phase high-performance liquid chromatography (RP-HPLC) system (Waters) equipped with a reverse phase C18 column (Zorbax, 300 SB-C18, 21.2 × 250 mm) and eluting with a linear gradient (flow rate 20 mL/min) using a binary buffer system of H₂O:MeCN:TFA (A: 95:5:0.1; B: 5:95:0.1). Fractions were collected at intervals of 0.3 minutes and characterized ESI-LCMS. Purity was determined by RP-UPLC at 214 nm, and fractions with purities >95% were pooled and lyophilized. The final lyophilized products were used in further experiments.

**Conjugation protocol for assembly of conjugates of peptides and AMPK activators:** The pure peptide and the pure thiopyridyl-activated AMPK activator conjugate was dissolved in a binary solvent system (A: DMF; 6 M Guanidine, 1.5 M Imidazole in H2O at pH = 8) (ratio 7:1) and agitated for at least 2 hours. The crude reaction mixture was monitored by analytical RP-UPLC and ESI-LCMS. Upon completion, the reaction mixture was diluted with buffer A and buffer B and purified directly using RP-HPLC eluting with a linear gradient.

**Desalting:** All peptides were desalted prior to biological experiments. Desalting was performed by consecutively re-dissolving the peptide or the conjugate of a peptide and an AMPK activator in dilute aqueous 0.01 M HCI followed by lyophilization, repeated 3 times. The purity of the peptide or the conjugate was monitored by RP-UPLC and ESI-LCMS before being used for in vivo or in vitro experiments.

**2-(pyridin-2-yldisulfaneyl)ethan-1-amine hydrochloride.** In a dry schlenk round-bottomed flask equipped with a magnetic stirring bar and under N₂-atmosphere, 2'-aldrithiol (5.00 g, 22.70 mmol, 3.0 equiv.) was dissolved in dry MeOH (20 mL) followed by addition of cysteamine hydrochloride (859.5 mg, 7.57 mmol, 1.0 equiv.). The reaction was stirred for 2 hours at room temperature and subsequently concentrated *in vacuo.* The crude yellow oil was purified by either silica gel flash column chromatography (CH₂Cl₂:MeOH 17:3) or redissolved in MeOH and precipitated with cold ether to afford 2-(pyridin-2-yldi-sulfaneyl)ethan-1-amine hydrochloride as a white crystalline (1.39 g, >95 %). R*_{f}* = 0.22; UPLC/MS (ESI): m/z calcd. for C₇H₁₀N₂S₂ [M+H]⁺ = 187.0, found 187.2; ¹H NMR (400 MHz, DMSO-*d*₆) δ 8.51 (dt, J = 4.9, 1.4 Hz, 1H), 8.23 (s, 3H), 7.84 (td, J = 7.7, 1.9 Hz, 1H), 7.78 - 7.72 (m, 1H), 7.30 (ddd, J = 7.4, 4.8, 1.1 Hz, 1H), 3.14-3.06 (m, 4H); ¹³C NMR (101 MHz, DMSO) δ 158.05, 149.83, 137.91, 121.64, 120.08, 37.67, 34.79..

**5-amino-1-((3a*R*,4*R*,6*R*,6a*R*)-6-(hydroxymethyl)-2,2-dimethylte-trahydrofuro[3,4-*d*][1,3]dioxol-4-yl)-1*H*-imidazole-4-carboxamide.** In a flame-dried schlenk round bottomed flask equipped with a magnetic stirring bar, Perchloric acid (70 %, 2.38 mL, 27.69 mmol, 1.3 equiv.) was added to a stirring suspension of Aicar (5.50 g, 21.3 mmol, 1.0 equiv) in dry acetone (360 mL). After stirring for 3h at room temperature, the reaction was quenched by dropwise addition of ammonium hydroxide (4.04 mL, 29.83 mmol, 1.4 equiv.) at 0 °C. The solid obtained was filtered and dried to afford 5-amino-1-((3aR,4R,6R,6aR)-6-(hydroxymethyl)-2,2-dimethyltetrahydrofuro[3, 4-d][1,3] dioxol-4-yl)-1H-imidazole-4-carboxamide as a white crystalline (4.26 g, 67 %). Purity >95 % (HPLC, 0-50B in 20 min), R*ₜ* = 16.60 min; UPLC/MS (ESI): m/z calcd. for C₁₂H₁₉N₄O₅ [M+H]⁺ = 299.1, found 299.4; ¹H NMR (400 MHz, DMSO-*d*₆) δ 7.37 (s, 1H), 6.74 (d, J = 50.9 Hz, 2H), 5.93 (s, 2H), 5.75 (d, J = 3.6 Hz, 1H), 5.27 (t, J = 4.9 Hz, 1H), 5.08 (dd, J = 6.3, 3.7 Hz, 1H), 4.87 (dd, J = 6.4, 2.9 Hz, 1H), 4.10 (q, J = 3.7 Hz, 1H), 3.52 (tq, J = 11.7, 6.1, 4.7 Hz, 2H), 3.31 (s, 1H), 1.53 (s, 3H), 1.32 (s, 3H); ¹³C NMR (101 MHz, DMSO) δ 166.60, 142.75, 128.16, 113.38, 112.82, 88.58, 84.97, 82.31, 80.53, 61.00, 26.96, 25.20.

**((3aR,4R,6R,6aR)-6-(5-amino-4-carbamoyl-1H-imidazol-1-yl)-2,2-dimethyltetrahydrofuro[3,4-d][1,3]dioxol-4-yl)methyl (2-(pyridin-2-yldisulfaneyl)ethyl)carbamate.** In a flame-dried schlenk round-bottomed flask equipped with a magnetic stirring bar and under N₂-atmosphere, 5-amino-1-((3a*R*,4*R*,6*R*,6a*R*)-6-(hydroxymethyl)-2,2-dimethyltetrahydrofuro[3,4-*d*][1,3]di-oxol-4-yl)-1*H*-imidazole-4-carboxamide (600.0 mg, 2.01 mmol, 1.0 equiv.) was dissolved in dry DMF (20 mL) followed by addition of *N*,*N*-Carbonyldiimidazole (391.0 mg, 2.41 mmol, 1.2 equiv.) and dry pyridine (488 µL, 6.03 mmol, 3.0 equiv.). The reaction was continuously monitored by UPLC-MS. Upon full conversion to the activated carbamate, 2-(pyridin-2-yldisulfaneyl)ethan-1-amine hydrochloride (468.4 mg, 3.02 mmol, 1.5 equiv.) was added and the reaction heated to 45 °C with an oil bath and stirred for 22h. Compound 2-(pyridin-2-yldisulfaneyl)ethan-1-amine hydrochloride was solubilized as the reaction progressed. Upon completion (monitored by UPLC-MS), the reaction was transferred to a separatory funnel with CH₂Cl₂ (75 mL) and washed with water (3×50 ml) and brine (2× 50 mL). The organic layer was dried over MgSO₄ and concentrated *in vacuo.* The crude oil was purified by purified by silica gel flash column chromatography using a gradient(CH₂Cl₂ -> CH₂Cl₂:MeOH 19:1 -> CH₂Cl₂:MeOH 9:1) to afford ((3aR,4R,6R,6aR)-6-(5-amino-4-carbamoyl-1H-imidazol-1-yl)-2,2-dimethylte-trahydrofuro[3,4-d][1,3]dioxol-4-yl)methyl (2-(pyridin-2-yldisulfaneyl)ethyl) carbamate as a white crystalline solid (916 mg, 89 %); Purity >95 % (HPLC, 0-50B in 20 min), R*ₜ* = 16.60 min; UPLC/MS (ESI): m/z calcd. for C₂₀H₂₇N₆O₆S₂ [M+H]⁺ = 511.1, found 511.2; ¹H NMR (400 MHz, DMSO-*d*₆) δ 8.51 ― 8.28 (m, 1H), 7.86 ― 7.73 (m, 2H), 7.51 (t, *J* = 5.7 Hz, 1H), 7.39 (s, 1H), 7.24 (ddd, *J* = 7.3, 4.8, 1.2 Hz, 1H), 6.76 (d, *J* = 31.7 Hz, 2H), 5.90 (s, 2H), 5.78 (d, *J* = 3.5 Hz, 1H), 5.17 (dd, *J* = 6.3, 3.5 Hz, 1H), 4.84 (dd, *J* = 6.3, 3.0 Hz, 1H), 4.24 (q, *J* = 4.4 Hz, 1H), 4.11 ― 3.97 (m, 2H), 3.28 (q, *J* = 6.5 Hz, 2H), 2.90 (t, *J* = 6.1 Hz, 2H), 2.52 (d, *J* = 2.3 Hz, 1H), 1.53 (s, 3H), 1.32 (s, 3H). ¹³C NMR (101 MHz, DMSO) δ 166.42, 159.01, 155.61, 149.58, 142.90, 137.79, 127.51, 121.19, 119.29, 113.72, 112.47, 87.60, 82.49, 82.27, 80.52, 63.70, 37.53, 26.87, 25.19.

**(2*R*,3*S*,4*R*,5*R*)-5-(5-amino-4-carbamoyl-1*H*-imidazol-1-yl)-3,4-di-hydroxytetrahydrofur an-2-yl)me thyl (2-(pyridin-2-yldisulfaneyl)ethyl)carbamate.** In a 10 mL vial with a screw cap, ((3aR,4R,6R,6aR)-6-(5-amino-4-carbamoyl-1H-imidazol-1-yl)-2,2-dimethyl-tetrahydrofuro[3,4-d][1,3]dioxol-4-yl)methyl(2-(pyridin-2-yldisulfaneyl)ethyl)carbamate (80.0 mg, 0.16 mmol, 1.0 equiv.) was dissolved in a 1:3 mixture of MeCN and 1.0 M aqueous HCI and left at the shaker for 3h (Until HPLC and UPLC-MS showed full conversion). Subsequently, the reaction mixture was lyophilized to afford (2*R*,3*S*,4*R*,5*R*)-5-(5-amino-4-carbamoyl-1*H*-imidazol-1-yl)-3,4-dihydroxytetra-hydrofuran-2-yl)methyl(2-(pyridin-2-yldisulfaneyl)ethyl)carbamate as the dihydrochloride salt (84 mg, >95 %). Purity >95 % (HPLC, 0-50B in 20 min), R*ₜ* = 11.62 min; UPLC/MS (ESI): m/z calcd. for C₁₇H₂₃N₆O₆S₂ [M+H]⁺ = 471.1, found 471.4; ¹H NMR (600 MHz, DMSO-*d*₆) δ 8.82 (s, 1H), 8.46 (dd, *J* = 5.0, 1.8 Hz, 1H), 7.85 (td, *J* = 7.8, 1.9 Hz, 1H), 7.79 (d, *J* = 8.0 Hz, 1H), 7.60 (t, *J* = 5.8 Hz, 1H), 7.26 (ddd, *J* = 7.5, 4.8, 1.1 Hz, 1H), 5.78 (d, *J* = 4.8 Hz, 1H), 4.37 (t, *J* = 5.0 Hz, 1H), 4.19 (m, 2H), 4.03 (m, 1H), 3.29 (q, *J* = 6.4 Hz, 2H), 2.92 (m, 2H); ¹³C NMR (151 MHz, DMSO) δ 162.32, 161.07, 158.92, 155.79, 149.35, 142.90, 138.13, 127.63, 121.31, 119.51, 103.12, 88.46, 83.31, 73.73, 69.93, 63.80, 37.58.

**(3*S*,3a*S*,6*S*,6a*S*)-6-((5-([1,1'-biphenyl]-4-yl)-6-chloro-1*H*-imidazo[4,5-*b*]pyridin-2-yl)oxy)hexa hydrofuro[3,2-*b*]furan-3-yl (2-(pyridin-2-yldisulfaneyl)ethyl)carbamate.** In a flame-dried round-bottomed flask under Argon atmosphere, MK-8722 (12 mg, 0.027 mmol, 1.0 equiv.), *N,N*-carbonyl diimidazole (5.2 mg, 0.032 mmol, 1.2 equiv.) and dry pyridine (6.5 µL, 0.08 mmol, 3.0 equiv.) were dissolved in dry DMF (1 mL) and left on a shaker (500 rpm) overnight at ambient temperature. The formation of the activated carbamate was confirmed by UPLC-MS; UPLC/MS (ESI): m/z calcd. for C₂₈H₂₄ClN₅O₅ [M+2H]²⁺ = 272,6 found 272.4. Upon full conversion as monitored by UPLC-MS, 2-(pyridin-2-yldisulfaneyl)ethan-1-amine hydrochloride (7.48 mg, 0.040 mmol, 1.5 equiv.) in dry DMF (1 mL) was added via a syringe and the temperature increased to 50 °C and left overnight. The reaction mixture was diluted with H₂O:MeCN (1:1) and purified by preparative HPLC (eluting with a gradient from 0-100% B, over 20 mL/min) followed by lyophilization affording (3S,3aS,6S,6aS)-6-((5-([1,1'-biphenyl]-4-yl)-6-chloro-1H-imidazo[4,5-b]pyridin-2-yl)oxy)hexahydrofuro[3,2-b]furan-3-yl (2-(pyridin-2-yldisul-faneyl)ethyl)carbamate as a white solid (11.6 mg, 66 %); Purity >95 % (HPLC), R_{T} = 16.77 min; UPLC-MS; UPLC/MS (ESI): m/z calcd. for C₃₂H₃₀ClN₅O₅S₂ [M+2H]²⁺ = 331,6 found 331.5; ¹H NMR (600 MHz, DMSO-*d*₆) δ 8.49 ― 8.43 (m, 1H), 7.95 (s, 1H), 7.83 (td, *J* = 7.7, 1.9 Hz, 1H), 7.81 ― 7.72 (m, 7H), 7.59 (t, *J* = 5.7 Hz, 1H), 7.50 (t, *J* = 7.7 Hz, 2H), 7.43 ― 7.37 (m, 1H), 7.32 ― 7.21 (m, 1H), 5.48 (q, *J* = 5.9 Hz, 1H), 4.94 (td, *J* = 6.9, 5.4 Hz, 1H), 4.87 (t, *J* = 5.2 Hz, 1H), 4.65 (t, *J* = 5.2 Hz, 1H), 4.13 (dd, *J* = 9.5, 6.1 Hz, 1H), 3.93 (ddd, *J* = 16.9, 9.2, 6.3 Hz, 2H), 3.68 (dd, *J* = 8.8, 7.3 Hz, 1H), 3.35 ― 3.22 (m, 2H), 2.92 (t, *J* = 6.8 Hz, 2H); ¹³C NMR (151 MHz, DMSO) δ 160.21, 159.06, 155.38, 149.57, 146.90, 139.69, 139.63, 137.88, 137.85, 130.09, 129.01, 127.65, 126.70, 126.09, 121.79, 121.22, 119.31, 116.22, 114.30, 80.44, 80.07, 78.61, 73.33, 70.12, 69.79, 37.59.

**Preparation of GlucagonPen40/AICAR (Penicillamine linked):** A Glucagon peptide derivative was synthesized using the Fmoc protocol disclosed above and conjugated with a chemical linker derivatized AICAR analog. The chemical synthesis of the chemical linker derivatized AICAR was performed via the synthetic route disclosed in Fig. 15.

**Glucagon Pen40/MK-8722:** The conjugate was prepared using the Conjugation protocol disclosed above and by the chemical reaction shown in Fig. 16, the chemical reaction being performed in 6M guanidine, 1.5M imidazole buffer at room temperature for 2 hours. RP-UPLC and ESI-LCMS analyses determined the purity to >95%.

**Example 2**: *In vivo* pharmacology studies in diet-induced obesity (DIO) mice.

## Claims

1. A conjugated molecule comprising a peptide displaying at least 0.1 % activity of native glucagon at the glucagon receptor, and an AMP-activated protein kinase (AMPK) activator, wherein the peptide is covalently bonded to the AMPK activator either directly or through a linker.

2. The conjugated molecule according to claim 1, wherein the peptide is of the glucagon-superfamily.

3. The conjugated molecule according to claim 1 or 2, wherein the peptide has at least 80% amino acid sequence identity to SEQ ID NO:1.

4. The conjugated molecule according to any one of the preceding claims, wherein the peptide consists of at least 10 amino acids and no more than 60 amino acids.

5. The conjugated molecule according to any one of the preceding claims, wherein the AMPK activator is covalently bonded to the peptide via a cleavable chemical linker, the cleavable chemical linker being selected from acid-cleavable linkers, enzyme-cleavable linkers, peptide-cleavable linkers, and linkers comprising a disulfide group.

6. The conjugated molecule according to claim 5, wherein the chemical linker has the formula R₁-R₃-S-S-R₄-R₅-NH-CO-R₂, wherein R₁ is the peptide, R₂ the AMPK activator, R₃ is optional and when present is selected from C(CH₃)₂, CH₂-CH₂, or CH₂, bonded to a side chain of the peptide or to a carbon atom of the backbone chain of the peptide, R₄ is (CH₂)ₙ or C₆H₄, R₅ is optional and when present is selected from C(CH₃)₂, CH-CH₃, CH₂-CH₂, or CH₂, and n is 1, 2, 3 or 4.

7. The conjugated molecule according to claims 1 to 4, wherein the AMPK activator is covalently bonded to the peptide via a non-cleavable linker, wherein the non-cleavable linker is selected from polyethylene glycol linkers, carbon linkers, SMCC and mc with conjugation chemistries of maleimides, ethers, amides, triazoles, disulfide, and thioether.

8. The conjugated molecule according to any one of the preceding claims, wherein the AMPK activator is selected from 5-Aminoimidazole-4-carboxamide 1-β-D-ribofuranoside (AICAR), (3R,3aR,6R,6aR)-6-((6-([1,1'-biphenyl]-4-yl)-7-chloro-3*H*-imidazo[4,5-b]pyridin-2-yl)oxy)hexahydrofuro[3,2-*b*]furan-3-ol (MK-8722), (3*R*,3a*R*,6*R*,6a*R*)-6-((6-chloro-5-(4-(1-(hydroxymethyl)cyclopropyl)phenyl)-1*H*-benzo[*d*]imidazol-2-yl)oxy)hexahydrofuro[3,2-*b*]furan-3-ol (PF-739), 6-Chloro-5-[4-(1-hydroxycyclobutyl)phenyl]-1H-indole-3-carboxylic acid (PF-06409577), 1,1-Dimethylbiguanide hydrochloride (metformin), 4-Hydroxy-3-(2'-hydroxybiphenyl-4-yl)-6-oxo-6,7-dihydrothieno[2,3-b]pyridine-5-carbonitrile (A-769662), 2-Chloro-5-[[5-[[5-(4,5-Dimethyl-2-nitrophenyl)-2-furanyl]methylene]-4,5-dihydro-4-oxo-2-thiazolyl]amino]benzoic acid (PT-1), 2-[[2-(2-Bromo-4-methylphenoxy)ethyl]thio]-pyrimidine (ZLN024), 2-[[4-(Diethylamino)-2-hydroxyphenyl]methylene]hydrazide-4-pyridinecarboxylic acid (RSVA-405), and analogues thereof.

9. The conjugated molecule according to any one of the preceding claims for use in therapy.

10. The conjugated molecule according to any one of the preceding claims for use in the treatment of obesity, type 2 diabetes, hyperinsulinemia, insulin resistance, impaired glucose tolerance, hypercholesterolemia, non-alcoholic fatty liver disease (NAFLD), non-alcoholic steatohepatitis (NASH) and dyslipidemia.

11. A pharmaceutical composition comprising the conjugated molecule according to any one of the claims 1 to 8 or a pharmaceutically acceptable salt thereof and a pharmaceutically acceptable carrier.

12. A method of reducing body weight of a mammal comprising administering the conjugated molecule according to any one of claims 1 to 9 or the pharmaceutical composition according to claim 11 to the mammal.

13. A non-therapeutic treatment of a mammal for reducing body weight comprising orally administering the conjugated molecule according to any one of claims 1 to 8 or the pharmaceutical composition according to claim 11 to the mammal.

14. The non-therapeutic treatment of a mammal for reducing body weight according to claim 13, wherein the mammal has a non-pathogenic body mass index (BMI).

15. A non-therapeutic treatment of a mammal for reducing plasma cholesterol comprising orally administering the conjugated molecule according to any one of claims 1 to 8 or the pharmaceutical composition according to claim 11 to the mammal.
